# EUROPEAN PATENT APPLICATION

(11) **EP 4 470 529 A1**
(43) Date of publication of application: **04.12.2024**
(21) Application number: 23747253.5
(22) Date of filing: 19.01.2023
(51) Int. Cl.: A61K 9/16, A61K 47/38, A61K 47/22, A61K 9/00, A61K 31/365, A61P 43/00

(54) **GRANULES COMPRISING PROGERININ, AND SACHETS USING SAME**

(30) Priority: 25.01.2022 KR 20220010690
(71) Applicant: PRG S&Tech Inc., Busan 46274 (KR)
(72) Inventor: PARK, Hong Gyun, Goyang-si Gyeonggi-do 10543 (KR)
(74) Representative: Mewburn Ellis LLP
(86) International application number: PCT/KR2023/000926
(87) International publication number: WO 2023/146211

(57) **Abstract**

The present invention relates to granules comprising Progerinin, capsules or sachets using same, and a preparation method therefor. More specifically, in order to develop a formulation that is advantageous when applying, to patients, Progerinin, which is a poorly water-soluble drug having a Hutchinson-Gilford progeria treatment effect, Progerinin granules comprising a specific water-soluble polymer, solubility enhancer and excipient at an optimum composition ratio have been developed and oral solid preparations such as capsules or sachets have been developed using the granules, such that dispersion stability and bioavailability of Progerinin can be improved and storage, transport and patient drug compliance can also be improved.

## Description

### Technical Field

The present disclosure relates to granules including progerinin which is a poorly soluble drug, a capsule or sachet using the same, and a preparation method thereof.

### Background Art

Many useful drugs are hydrophobic with low solubility in an aqueous medium, making it difficult to formulate as a suspension in an aqueous vehicle. In particular, due to this property, wetting agents are often required to promote suspension of hydrophobic drug particles in aqueous media. It is known that surface-active wetting agents (i.e., surfactants) such as sodium lauryl sulfate reduce the interfacial tension between the drug particles and the suspension vehicle to allow suspension vehicles to penetrate into drug aggregates and/or pores on the drug particle, thereby increasing suspension properties of hydrophobic drugs in the aqueous medium, at least partially. However, besides beneficial effects of drug-suspension, the use of surfactants in the suspension may also lead to undesirable consequences of soluble/dissolved free drugs.

Since soluble/dissolved drugs are susceptible to chemical degradation and/or interactions with other components, suspensions containing free drugs may be chemically unstable. Another undesirable consequence of using relatively high amounts of surfactant to promote suspension of low soluble drugs is that because surfactants stabilize bubbles, accompanying air may tend to remain trapped during homogenization or shaking of these suspensions. Since this trapped air changes with the stirring force, duration of the stirring, and an elapsed time of the stirring, the volume of the suspension changes, making it difficult or impossible to administer at a uniform dose over time.

If a low water-soluble drug is administered as a suspension, it is desirable that the suspension shows gentle sedimentation to provide adequate uniformity in the dose. Conversely, if rapid sedimentation occurs, as the case of the vehicle, the suspension must be shaken before each administration in order to achieve uniformity in the dose. If other factors (such as drug particle size, uniformity, and density) are equivalent, a sinking rate of drug particles decreases as a viscosity of a particular suspension vehicle increases. Therefore, it is desirable that the suspension be moderately viscous to inhibit or slow down the sedimentation of drug particles. However, while this increased viscosity promotes physical stability, it also makes it difficult to pour or administer the suspension.

On the other hand, progerinin, which has a structure represented by the following Chemical Formula 1, has shown excellent effects of suppressing progerin expression and effects of inhibiting binding between progerin and lamin A, and, as a drug exhibiting effects of prolonging a survival period of progeria-induced animal model, it may be used as a pharmaceutical composition for preventing or treating progeria.

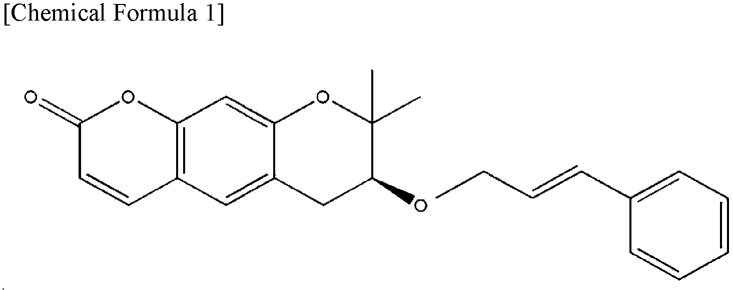

The compound name of the progerinin is (7S)-(+)-8,8-dimethyl-7-(3-phenylallyloxy)-7,8-dihydro-6H-pyrano[3,2-g]chromen-2-one (named 'SLC-D011').

Progerinin, a drug that has been shown to be effective in progeria, is a BCS class II molecule exhibiting high apparent permeability and low solubility in aqueous media according to the criteria of the Biopharmaceutical Classification System (BCS).

Due to the low water solubility of progerinin, their therapeutic applications are very limited. To overcome these limitations, a number of methods have been studied, such as the use of polymeric nanoparticles, solid lipid nanoparticles, autologous emulsification drug delivery systems, nanoemulsions, liposomes, nanosuspensions, and nanofibers. Thereamong, the nanosuspension is a colloidal dispersion of stabilized drug particles in the presence of polymers, surfactants, or both. The nanosuspension is used to deliver drug substances that exhibit low water solubility and lipid solubility. The small particles in the nanosuspension provide a very large drug surface area, thereby increasing a dissolution rate of insoluble drugs. Consequently, BCS class II and IV compounds may exhibit enhanced bioavailability, rapid activity, and other desirable biopharmaceutical effects.

Under these circumstances, the inventors were able to conduct animal experiments using a mixture of monoolein and tricaprin, which are oil-based solutions, but due to the nature of the disease, it was found that the use of an oil-dissolved formulation is impossible as high doses and long-term intake are required, recognizing a need for development of a new formulation that allows a long-term intake. In particular, the above-mentioned progerinin is poorly soluble to the extent that its solubility in water is close to zero, and the solubility is very low even in existing ingestible solvents, such that it is necessary to develop a formulation that enables long-term intake and body absorption through the introduction of new technology.

In addition, in Korean Patent Application No. 10-2020-0113071 (filed on September 4, 2020), the present inventor optimized the particle diameter of a progerinin drug with excellent bioabsorption efficiency and selected water-soluble polymers and excipients for the preparation of nanosuspensions to provide a stability-enhanced oral nanosuspension preparation containing progerinin with excellent dispersibility or uniformity and improved bioavailability, but there were inconveniences in terms of storage, transportation, and patient administration.

Therefore, there is a need to develop improved formulations to make oral nanosuspension preparations advantageous in terms of storage, transportation, and patient administration.

### Disclosure of the Invention

### Technical Goals

An object of the present disclosure is to provide granules including progerinin with excellent bioavailability and a preparation method thereof.

Another object of the present disclosure is to provide a capsule or sachet using granules including progerinin in order to provide an improved formulation with excellent bioavailability to be more advantageous in terms of storage, transportation, and patient administration, and a preparation method thereof.

### Technical Solutions

To achieve the above objects, the present disclosure provides a granule including progerinin, including: (a) 20 to 40 wt% of progerinin represented by the following Chemical Formula 1; (b) 5 to 15 wt% of hydroxypropyl methylcellulose (HPMC); (c) 1 to 10 wt% of D-α-tocopherol polyethylene glycol succinate (TPGS); and (d) a residual amount of additives, wherein the progerinin is drug particles formed via wet type ball milling with an average particle diameter (D50) of 100 nm to 300 nm.

In addition, the present disclosure provides a capsule or sachet including the granule.

In addition, the present disclosure provides a method of preparing a granule including progerinin, including: (1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension; (2) subjecting the suspension to wet type ball milling to prepare a nanosuspension; and (3) mixing the nanosuspension with one or more additives selected from the group consisting of excipients, binders, disintegrants, and lubricants, followed by granulating in a fluid bed granulator, wherein the progerinin in the nanosuspension of the step (2) is in the form of particles with an average particle diameter (D50) of 100 nm to 300 nm.

### Advantageous Effects

In order to develop a formulation that is advantageous for patient application of progerinin which is a poorly soluble drug effective in treating progeria, according to the present disclosure, progerinin granules including specific water-soluble polymers, dissolution enhancers, and excipients in an optimal composition ratio were developed, and, by developing oral solid preparations such as capsules or sachets using the granules, it was possible to improve the dispersion stability and bioavailability of progerinin, as well as storage, transportation, and patient compliance.

### Brief Description of Drawings

FIG. 1 shows results of pharmacokinetic analysis of a progerinin suspension.
FIG. 2 shows a result of measuring a solubility of a solid dispersion prepared via hot melt extrusion (HMT).
FIG. 3 shows a result of measuring a solubility of a solid dispersion prepared via a hot melt extrusion process with polymers in various ratios (1:1).
FIG. 4 shows a result of pharmacokinetic analysis on a solid dispersion prepared via a hot melt extrusion process.
FIG. 5 shows results of measuring a solubility for an amorphous solid dispersion via a spray drying process (A: pH 1.2, B: pH 6.8).
FIG. 6 shows a result of pharmacokinetic analysis on a solid dispersion prepared via a spray drying process.
FIG. 7 shows images of a nanosuspension prepared via wet type ball milling with beads (top) and a result of measuring an average particle diameter of a progerinin drug (bottom).
FIG. 8 shows a process of preparing granule, capsule, and sachet formulations of progerinin according to the present disclosure.

### Best Mode for Carrying Out the Invention

Hereinafter, the present disclosure will be described in detail.

The present inventor had prepared a nanosuspension of progerinin to improve the bioavailability of progerinin which is a poorly soluble drug, but when developed as an actual drug, it is required to consider aspects of storage and transportation issues as well as patient compliance. Therefore, the present inventor has completed the present disclosure by developing an oral solid formulation such as a capsule or a sachet using the nanosuspension.

The present disclosure provides a granule including progerinin, including (a) 20 to 40 wt% of progerinin represented by the following Chemical Formula 1; (b) 5 to 15 wt% of hydroxypropyl methylcellulose (HPMC); (c) 1 to 10 wt% of D-α-tocopherol polyethylene glycol succinate (TPGS); and (d) a residual amount of additives, wherein the progerinin is drug particles formed via wet type ball milling with an average particle diameter (D50) of 100 nm to 300 nm:

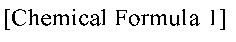

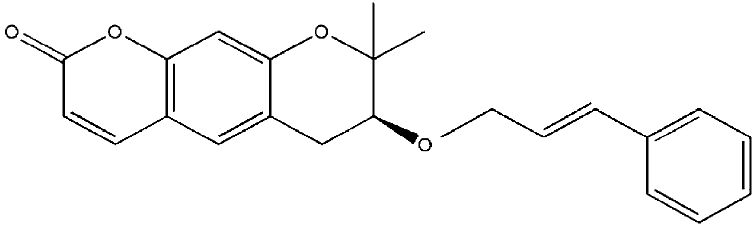

The progerinin is a decursin derivative which is (7S)-(+)-8,8-dimethyl-7-(3-phenylallyloxy)-7,8-dihydro-6H-pyrano[3,2-g]chromen-2-one. In the present disclosure, progerinin may also be named 'SLC-D011'.

Progerinin has shown excellent effects of suppressing progerin expression and effects of inhibiting binding between progerin and lamin A and may be used as a pharmaceutical composition for preventing or treating progeria, an age-related disease, as a drug that has an effect of prolonging a survival period in progeria-induced animal models. While progerinin is poorly soluble to the extent that its solubility in water is close to zero (0), according to the present disclosure, provided is an oral solid formulation for enhancing solubility, which allows for long-term intake and body absorption and takes into account storage and transportation issues and patient compliance aspects.

The hydroxypropyl methylcellulose (HPMC) as a polymeric suspension agent is a component which helps dispersion of progerinin and other components in a solution, wherein, if the polymeric suspension agent is included below the above content range, the sinking rate of the suspension becomes poor, and if the polymeric suspension agent is included above the range, preparation may become challenging due to difficulty in stirring. In particular, if the polymeric suspension agent is included above the range, the dispersibility of the suspension is lowered due to poor uniformity in the stirring, which may result in an increase in the volume of defective products with insufficiency in the content during production of the product.

The D-α-tocopherol polyethylene glycol succinate (TPGS) is a solubility enhancer, which is a component that enhances solubility of the poorly soluble drug, wherein, if the solubility enhancer is included below the range, the solubility is lowered while the toxicity tolerance limit may be exceeded if included above the range, such that it is desirable to use within the above range.

It is desirable for progerinin, a poorly soluble drug in the suspension, to have an average particle diameter (D50) of 100 nm to 300 nm. Preferably, the average particle diameter may be 100 nm to 200 nm. With the average particle diameter within the above range, the solubility is the highest, and nanoparticles have a larger surface area to be dissolved more easily, thereby maximizing the bioavailability or bioabsorption efficiency. Moreover, for the preparation of nanosuspensions having the average particle diameter, it may be prepared via wet type ball milling using wet grinding apparatuses such as an agitator bead mill or a dyno-mill.

In addition, the pH of the nanosuspension of the present disclosure may be 5.5 to 8.5, preferably 6.0 to 7.0, and the nanosuspension may have a viscosity of 2 to 8.5 mPa.s, preferably 7 to 8.5 mPa.s.

In addition, the nanosuspension of the present disclosure may additionally include preservatives, pH adjusters, colorants, or the like.

The preservative refers to a pharmaceutically acceptable substance that prevents microbial proliferation or degradation by undesired chemical changes. Potassium sorbate may be used as the preservative, but is not limited thereto.

The pH adjuster refers to a substance that is used to adjust the pH of the suspension to a desired value and may be citric acid, sodium citrate, or ascorbic acid, but is not limited thereto.

The colorant is included to vary the color of the nanosuspension and may be used to prepare the nanosuspension having a desired color by being included in the formulation. Anything that is commonly used in the technical field of the present disclosure may be included herein without limitation.

The additives may be one or more additives selected from the group consisting of excipients, binders, disintegrants, and lubricants.

The excipient refers to a substance added for the purpose of giving a drug a suitable hardness or shape, or for the purpose of giving a certain dose and weight in the case of a small amount of main ingredients so as to make it a size that is easy to handle. The excipients may be, but are not limited to, one or more selected from the group consisting of mannitol, starch, microcrystalline cellulose, dextrin, sodium alginate, methylcellulose, sodium carboxymethylcellulose, lactose, glucose, fructose, sodium alginate, and hydroxypropyl starch.

The binder refers to a substance added for the purpose of combining the particles of an ingredient to have a certain size. The binders may be, but are not limited to, one or more selected from the group consisting of gelatin, gum arabic, glucose, ethanol, purified water, dextrin, glycerin, microcrystalline cellulose, methylcellulose, hydroxypropyl cellulose, hydroxypropyl methylcellulose, carboxymethylcellulose, sodium carboxymethylcellulose, and calcium carboxymethylcellulose.

The disintegrant refers to a substance added for the purpose of promoting disintegration of tablets, capsules, and granules in digestive juices. The disintegrants may be, but are not limited to, one or more selected from the group consisting of sodium croscarmellose, starch, hydroxypropyl starch, methylcellulose, sodium alginate, calcium citrate, sodium carboxymethylcellulose, calcium carboxymethylcellulose, silicic acid anhydride, sodium lauryl sulfate, carbonate, and dextran.

The lubricant refers to a substance that improves the fluidity of the granule and thus increases its chargeability. The lubricants may be, but are not limited to, one or more selected from the group consisting of calcium stearate, magnesium stearate, sodium stearate, stearic acid, hydrogenated vegetable oil, talc, kaolin, liquid paraffin, and magnesium oxide.

In addition, the present disclosure provides a capsule including the granule in a capsule.

In addition, the present disclosure provides a sachet including the granule, a sweetener, and a flavor.

The sweetener refers to a food additive that exhibits sweetness, but generally has a lower food caloric value, including both natural or synthetic, and may be used without particular limitation if the sweetener is one that may be used conventionally in the field of food and medicine, including sucralose.

The flavor includes a flavoring ingredient or composition commonly used in the food industry, whether it has a natural or synthetic origin, and the flavor may be used without particular limitation if it is conventionally used in the field of food and medicine, including grape flavor.

In addition, the present disclosure provides a method of preparing a granule including progerinin, including (1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension; (2) subjecting the suspension to wet type ball milling to prepare a nanosuspension; and (3) mixing the nanosuspension with one or more additives selected from the group consisting of excipients, binders, disintegrants, and lubricants, followed by granulating in a fluid bed granulator, wherein the progerinin in the nanosuspension of the step (2) is in the form of particles with an average particle diameter (D50) of 100 nm to 300 nm.

Since the role of each component and dose thereof are the same as mentioned above, no further explanation is given.

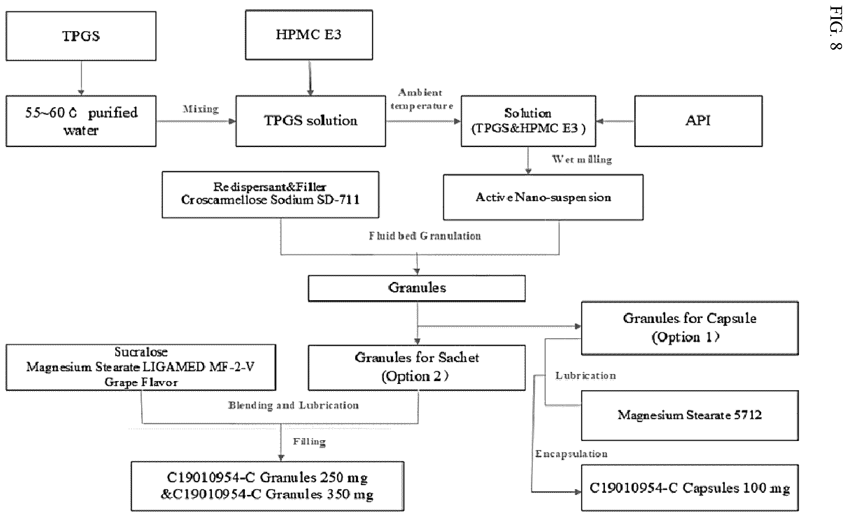

FIG. 8 is a flow diagram illustrating a preparation process of granule, capsule, and sachet formulations of progerinin according to the present disclosure.

First, in the present disclosure, the step (1) is a process of mixing micronized progerinin into a vehicle solution, specifically mixing hydroxypropyl methylcellulose (HPMC), which is a polymeric suspension agent, and D-α-tocopherol polyethylene glycol succinate (TPGS) in purified water. Here, the purified water may be used at a temperature of 50 to 70°C, preferably at 60°C. The coarse suspension thus mixed is then cooled at room temperature, followed by the next step to prepare the nanosuspension.

Next, the step (2) is a step of preparing the nanosuspension by wet type ball milling of the suspension of (1). Here, the wet type ball milling may be performed by means of a wet grinding apparatus, such as an agitator bead mill or a dyno-mill. It is desirable for ball milling conditions to proceed until the average particle diameter of the drug particles is between 100 nm and 300 nm. More preferably, the average particle diameter is 100 nm to 200 nm. Having the average particle size within the above range, the solubility becomes the most superior, and it is possible to maximize the bioavailability or bioabsorption efficiency of the drug.

The ball milling is wet type ball milling for the preparation of nanosuspensions, which is an important step in the preparation of progerinin nanosuspensions, and selection of suitable bead size, grinding medium, and API ratio in a chamber as well as temperature control of a grinding chamber may play an important role.

Specifically, during the wet type ball milling in the preparation of nanosuspensions, a suspension of the step (1) and zirconia beads with an average particle diameter of 0.1 to 1 mm may be used, preferably those of 0.02 to 0.04 mm may be used. Further, the suspension and zirconia beads of the step (1) may be used in a volume ratio of 1:1 to 1:5, but if it is possible to produce the drug to have the average particle diameter (D50) of 200 nm or less, the volume ratio may be changed appropriately according to conditions.

Next, the step (3) is a step of preparing a granule by mixing the nanosuspension prepared in the step (2) with one or more additives selected from the group consisting of excipients, binders, disintegrants, and lubricants, followed by granulating the mixture in a fluid bed granulator.

In addition, the present disclosure provides a method of preparing a capsule including progerinin, including (1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension; (2) subjecting the suspension to wet type ball milling to prepare a nanosuspension; (3) mixing the nanosuspension with one or more additives selected from the group consisting of excipients, binders, disintegrants, and lubricants, followed by granulating in a fluid bed granulator; and (4) filling the granulated granule in a capsule, wherein the progerinin in the nanosuspension of the step (2) is in the form of particles with an average particle diameter (D50) of 100 nm to 300 nm.

In addition, the present disclosure provides a method of preparing a sachet including progerinin, including (1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension; (2) subjecting the suspension to wet type ball milling to prepare a nanosuspension; (3) mixing the nanosuspension with one or more additives selected from the group consisting of excipients, binders, disintegrants, and lubricants, followed by granulating in a fluid bed granulator; and (4) adding a sweetener and a flavor to the granulated granule and then filling the granule into a sachet, wherein the progerinin in the nanosuspension of the step (2) is in the form of particles with an average particle diameter (D50) of 100 nm to 300 nm.

### Modes for Carrying Out the Invention

Hereinafter, to help the understanding of the present disclosure, example embodiments will be described in detail. However, the following example embodiments are provided to explain the present disclosure more completely to those with ordinary skill in the art and it is merely illustrative of the contents of the present disclosure, and therefore the scope of the present disclosure is not limited to the following example embodiments.

### <Example 1> Preparation of a lipid-based progerinin solution and limitations

In order to overcome the poor bioavailability of progerinin drugs, the inventors prepared a progerinin solution using a lipid-based preparation (monoolein:tricaprylin = 2:1) in a preclinical study. However, lipid-based preparations were determined to be unsuitable for use in clinical settings due to inadequate drug loading and high lipid intake expected with the proposed clinical dose. Limited attempts to produce amorphous solid dispersions (ASDs) have shown that there is a difficulty for the amorphous solid dispersions (ASDs) to be dispersed in aqueous media due to rapid crystallization. In addition, these lipid-based preparations are considered unsuitable for use in clinical settings due to inadequate drug loading and high lipid intake expected with the proposed clinical dose.

Accordingly, the inventors aimed to prepare progerinin, which is a poorly soluble drug, as a nanosuspension and implement the nanosuspension that is optimized for clinical use by selecting a vehicle composition that may enhance the stability of a drug.

Therefore, while the present inventors have determined that selection of stabilizers such as polymers and surfactants and a particle size in the suspension are important factors in the manufacture of nanosuspensions of poorly soluble drugs, of which the size of the drug particles in the drug has priority as an important factor in determining the bioabsorption efficiency, as shown in the experiments below, the average particle diameter of progerinin (SLC-D011) with excellent bioabsorption efficiency was selected, and polymers, surfactants, and preservatives were optimized for the production of nanosuspensions.

### <1-1> Determination of average particle diameter (D50) of progerinin drug

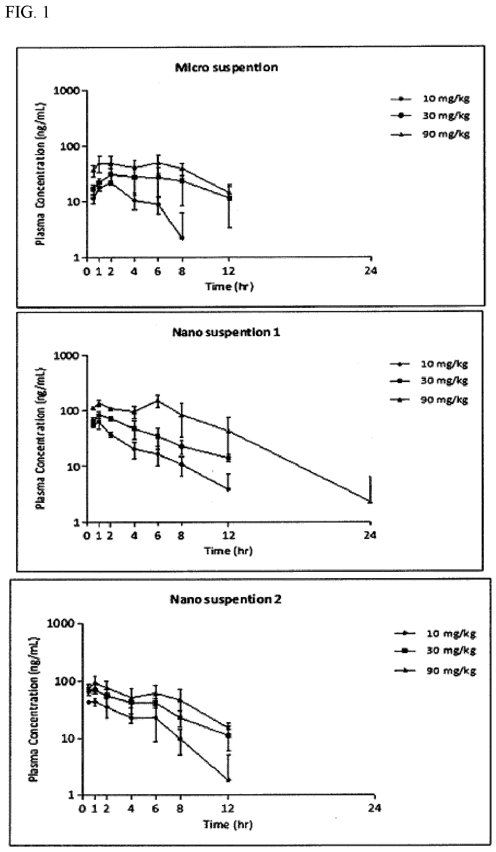

As mentioned above, the size of the drug particles in the drug is an important factor in determining the bioabsorption efficiency. Therefore, in order to design a diameter size that is highly bioabsorbable for the progerinin drug, the inventors used mice to analyze pharmacokinetics (PK) of compounds. Specifically, a microsuspension including micro-sized progerinin (D 50 = 1.5 mm), a nanosuspenstion 1 (Nano suspenstion 1 = 200 nm (D=50)) and a nanosuspenstion 2 (Nano suspenstion 2 = 350 nm (D=50)) including nano-sized progerinin were prepared. Here, the microsuspension was prepared using Vivapur power, and the two nanosuspensions were prepared using zirconia beads. The suspension prepared as described above was administered orally to mice at doses of 10 mg/kg, 30 mg/kg, and 100 mg/kg, and results were checked by analyzing the pharmacokinetics (PK) of the suspension in vivo (FIG. 1).

As a result of FIG. 1, it was determined that the bioabsorption of the drug is satisfied when the average particle size is 200 nm or less. Therefore, the inventors decided to develop in a suspension formulation including drug nanoparticles with an average particle diameter of 200 nm or less.

In order to devise a manufacturing method that can satisfy these formulations, the present inventors performed a process using hot melt extrusion, spray drying, ultra-high pressure homogenizer, and wet type ball milling using beads and attempted to select the most suitable method for preparing the progerinin nanosuspension among them.

### <1-2> Preparation of amorphous solid dispersions using hot melt extrusion (HME) process

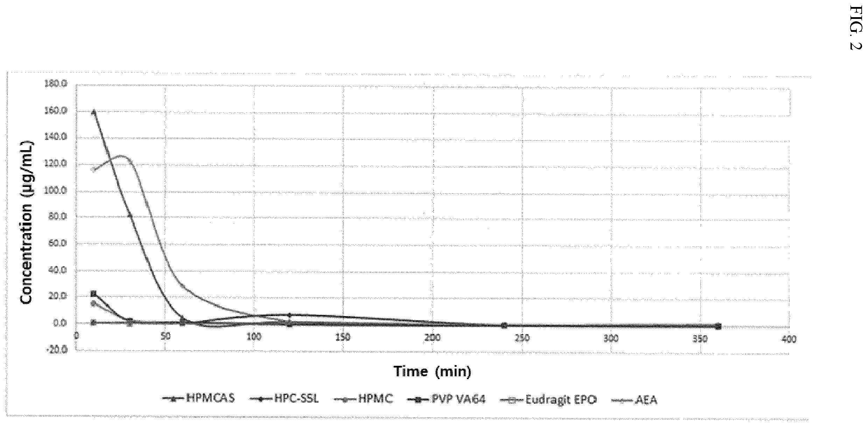

First, solid dispersions for various polymers (HPMCAS, HPC, HPMC, PVP VA64, Eudragit EPO, AEA) were prepared using the hot melt extrusion method. Here, the ratio of drug to polymer was set to 1:3 to prepare the mixture. Solubility tests were performed on a solid dispersion equivalent to 100 mg of progerinin with 300 mL of pH 1.2 buffer (Eudragit EPO, AEA) or 300 mL of pH 6.8 buffer (HPMCAS, HPC, HPMC, PVP VA64) as an eluate (FIGS. 2 to 4). The elution conditions are as follows: paddle method, temperature: 37°C, rotation rate: 150 rpm

As a result of FIG. 2, it was found that the solubility was improved the highest when HPMCAS was used as a polymer.

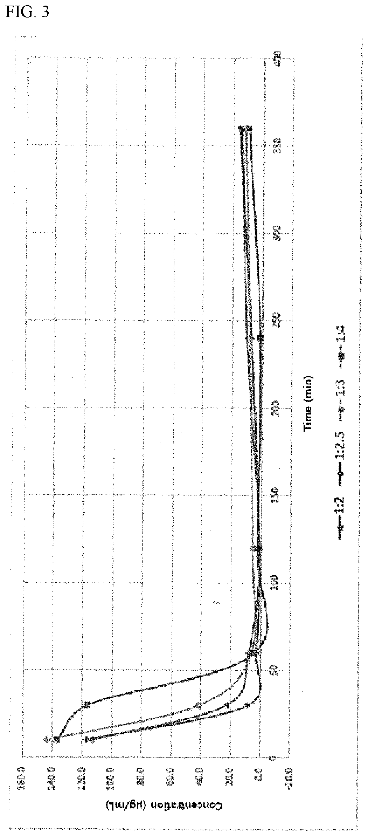

Next, the HPMCAS with the most improved solubility was re-selected and the solid dispersion was prepared in various ratios (1:2, 1:2.5, 1:3, 1:4). Solubility test was conducted on a solid dispersion equivalent to 100 mg of progerinin with 300 mL of pH 6.8 buffer as an eluate (FIG. 3). The elution conditions are as follows: paddle method, temperature: 37°C, rotation rate: 150 rpm

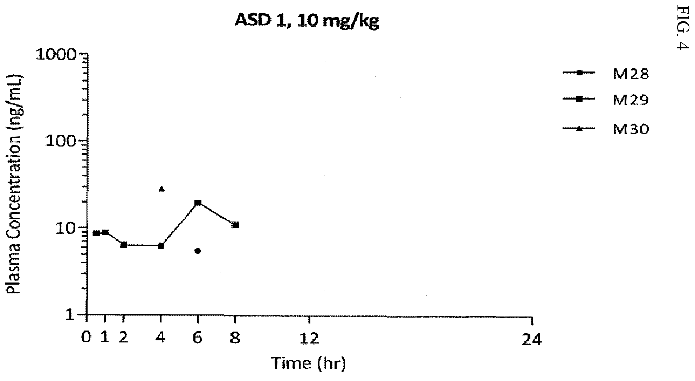

As a result of FIG. 3, it was found that the solubility was highest when the ratio of the drug to HPMCAS was 1:3. However, in fact, it was possible to obtain a light-yellow powder form through the HME method and it was found that it remained amorphous in a solid form. In addition, as a result of FIG. 4, the powder obtained by the HME method immediately settled by precipitation after contact with water, and when bioabsorption using mice was observed, there was almost no absorption into vivo when administered orally.

Therefore, it was determined that progerinin, which is the main component of the current drug, could not be developed in a formulation by the hot melt extrusion process.

### <1-3> Preparation of amorphous solid dispersions via spray drying process

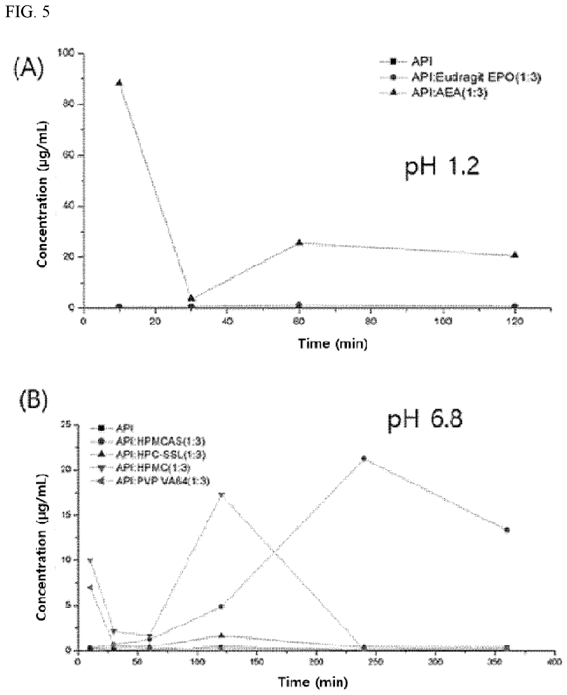

The polymer of the Example <1-2> was used, but an amorphous solid dispersion was prepared using the spray drying process. Solid dispersions for various polymers (HPMCAS, HPC, HPMC, PVP VA64, Eudragit EPO, AEA) were prepared via the spray drying process. At this time, the mixture was prepared by setting a ratio of drug and polymer to 1:3. Solubility test was conducted on a solid dispersion equivalent to 100 mg of progerinin with 300 mL of pH 1.2 buffer (Eudragit EPO, AEA) and 300 mL of pH 6.8 buffer (HPMCAS, HPC, HPMC, PVP VA64) as an eluate, respectively (FIG. 5). The elution conditions are as follows: paddle method, temperature: 37°C, rotation rate: 150 rpm

As a result of FIG. 5, it was found that the solubility of the polymer was improved the most at pH 1.2 and 6.8 when HPMCAS was used as the polymer in the same way as the solid dispersant prepared by the hot melt extrusion process.

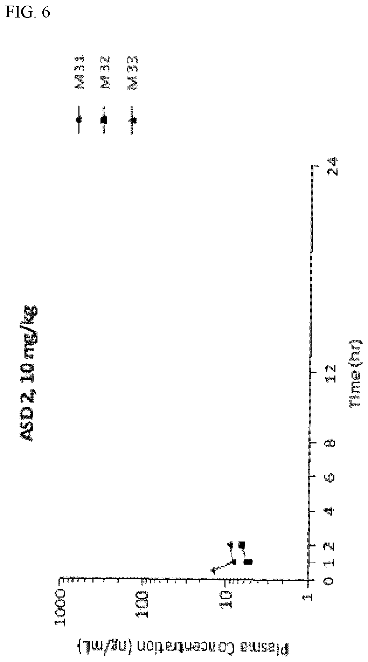

However, when bioabsorption (PK analysis) using mice was identified, absorption into vivo barely took place when administered orally, similar to HME (FIG. 6). In addition, the stability of other polymers under high temperature and high humidity for 2 weeks showed unsatisfactory results as the recrystallization of progerinin particles was observed (Table 1).

**TABLE 1**

| SD formu lation | Initial purity (%) | Storage condition | After 1 week | | | | | After 2 weeks | | | |
|---|---|---|---|---|---|---|---|---|---|---|---|
| | | | Appea rance | Tg by mDS C (°C) | XRPD | HPLC results | | XRPD | Tg by mDS C (°C) | HPLC results | |
| | | | | | | Drug load (%) | Purity (%) | | | Drug load (%) | Purity (%) |
| Crysta lline API | 99.85 | 25°C /60%RH, open | White powde r | N/A | Unchan ged | N/A | 99.85 | Unchan ged | N/A | N/A | 99.85 |
| | | 40°C /75%RH, open | White powde r | N/A | Unchan ged | N/A | 99.85 | Unchan ged | N/A | N/A | 99.85 |
| PVP VA64 | 96.36 | 25°C /60%RH, open | White powde r | 79.19 | Amorp hous | 19.9 | 96.56 | Amorp hous | 83.14 | 25.6 | 96.27 |
| | | 40°C /75%RH, open | White powde r | N/A | Crystall ine | 21.3 | 96.36 | Crystall ine | N/A | 22.5 | 96.39 |
| HPM C ASLF | 92.63 | 25°C /60%RH, open | White powde r | 67.25 | Amorp hous | 20.8 | 92.36 | Amorp hous | 67.82 | 22.5 | 92.04 |
| | | 40°C /75%RH, open | White powde r | N/A | Crystall ine | 21.7 | 91.53 | Crystall ine | N/A | 23.4 | 91.62 |
| HPM C E3 | 80.72 | 25°C /60%RH, open | White powde r | 98.75 | Amorp hous | 18.0 | 79.41 | Amorp hous | 96.50 | 17.1 | 79.65 |
| | | 40°C/75 %RH, open | White powde r | N/A | Crystall ine | 18.2 | 78.60 | Crystall ine | N/A | 16.2 | 77.45 |

Therefore, it was determined that progerinin, which is the main component of the current drug, could not be developed in a formulation by the spray drying method.

### <1-4> Preparation of nanosuspensions using ultra-high pressure homogenizers

The inventors have prepared the nanosuspensions using an ultra-high pressure homogenizer as an alternative method to prepare the nanosuspension. Specifically, polymer (HPMC 3cp) and surfactant (TPGS) were dissolved in a 100 mL beaker containing distilled water, and then the drug (SLC-D011) was weighed and added to prepare the suspension by stirring for 2 hours. The nanosuspension was prepared by injecting the suspension into an ultra-high pressure homogenizer (pressure: 40000 psi, 1 hour).

**TABLE 2**

| | Initial | 3 Day |
|---|---|---|
| Z-average (nm) | 338.6 | 354.6 |
| PI | 0.076 | 0.278 |
| D (90%) (nm) | 517.1 | 893.9 |

Table 2 is a measurement of the particle diameter of the drug, identifying that the size of the particles increased after 3 days. Therefore, the preparation of nanosuspensions using ultra-high pressure homogenizers had the limitation that the particles increase over time. Again, this is not a suitable method for the preparation of progerinin nanosuspensions.

### <1-5> Preparation of nanosuspensions via wet type ball milling with beads

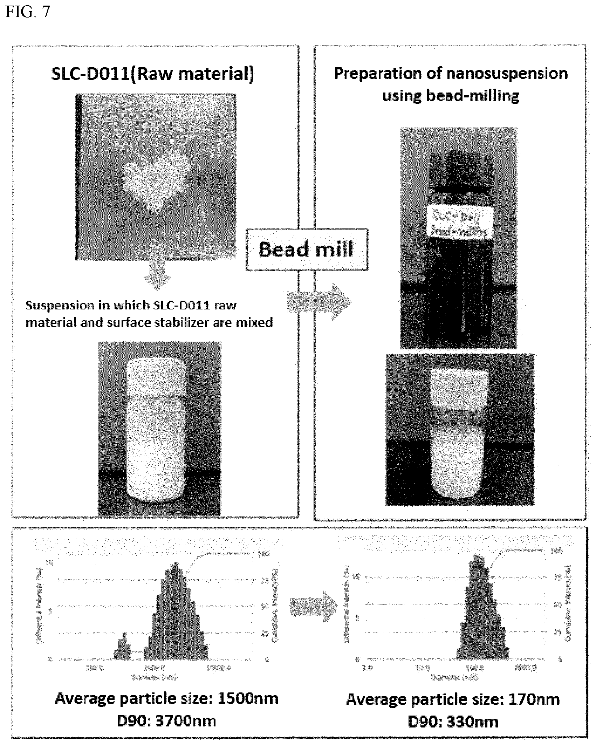

As an alternative method, the inventors prepared a nanosuspension using wet type ball milling using beads. Specifically, polymer (HPMC 3cp) and surfactant (TPGS) were dissolved in a 100 mL beaker containing distilled water, and then progerinin was weighed and added to prepare the suspension by stirring for 2 hours. Tubes with beads and suspensions added were mounted on DeltaVita^{®}, Netzsch (Zentrimix 380R) devices to prepare the nanosuspension (rate: 1200 rpm, temperature: -10°C, 6 hours). FIG. 7 shows the appearance (top) of the nanosuspension prepared by the preparation method and results (bottom) of measuring the average particle diameter.

As a result of FIG. 7, it was possible to prepare the nanosuspension including drug nanoparticles with an average particle diameter of 170 nm. In terms of the stability of nanosuspensions, it was found that the solubility was more excellent when the average particle diameter of the drug particles is 200 nm or less, and that the nanoparticles have a larger surface area and are more easily dissolved, thus maximizing the bioavailability.

Accordingly, the inventors determined that it was necessary to select a vehicle composition that is suitable for the preparation of nanosuspensions with an average particle diameter of 200 nm or less while preparing nanosuspensions using wet type ball milling using beads, and sought to select optimized polymers, surface stabilizing agents such as surfactants, and preservatives.

### <Example 2> Selection of optimized vehicle solutions

### <2-1> Preparation of progerinin nanosuspensions

### a) Preparation of suspensions (Step (1))

TPGS solution was prepared by dissolving TGPS in 60°C warm water and then cooling it to room temperature. HPMC E3 solution was prepared by dissolving HPMC E3 in 60°C warm water and then cooling it to room temperature. Then the two solutions were stirred and mixed thoroughly. To prepare a suspension, progerinin as an active pharmaceutical ingredient (API) was slowly filled in the mixed solution with continuous stirring.

### b) Nano milling (Step (2))

Parameters of the Agitator Bead Mill, a mixing and wet grinding equipment, were set appropriately to start the wet type ball milling, and a hydrostatic pump was adjusted to have the suspension circulated. The suspension coming from the Agitator Bead Mill was recirculated back into a suspension vessel while maintaining a preset value below 35°C. Wet type ball milling continued until PSD (D50 ≤ 200 nm) of the drug particles was achieved. When the target PSD (D50≤200nm) was accomplished, the pump was turned off, and the milling suspension line was separated from the suspension vessel.

### c) Preparation of nanosuspensions with enhanced stability (Step (3))

The suspension obtained by nano-milling was thoroughly mixed by adding potassium sorbate as a preservative. It was placed and filled into a sterilized glass bottle. The operation of filling the bottle was carried out with 100% visual inspection and checking of filling weight, after which the bottle was sealed with a rubber finish and an aluminum flip-off seal.

### <2-2> Screening of optimized vehicle solutions

The oral progerinin nanosuspension was prepared via the method in Example <2-1>. Specifically, Prototype 1 consists of 10.0 wt% of progerinin, 3.0 wt% of HPMC E3, 1.0 wt% of surfactant TPGS, and 0.2 wt% of potassium sorbate, and a suspension was prepared by mixing TPGS and HPMC solutions and then adding progerinin. The mixture prepared as described above was then mixed with 0.3 mm VHD ZrO beads in a volume ratio of 1:1 to 5, and the ball milling was performed. The temperature of the vessel was closely monitored during the preparation process. Furthermore, during milling, the particle size distribution was monitored until the target average particle size reached D50 ≤ 200 nm. Prototypes 2 to 5 were also prepared in the same way. The particle diameter, zeta potential, drug content, flowability, and viscosity of the nanosuspension prepared as described above were measured in the following methods.
(1) Measurement of particle diameter (D50): Approximately 2 µL of nanosuspension was dispersed in 1 mL of water and put into a sample tube, followed by measurement with the Zeta Potential & Particle Sizer (ZPPS) (Nicomp 380/ZLS, Nicomp), which is used for aqueous suspensions.
(2) Measurement of zeta potential: It is measurement of the electrical charge of the particle surface, indicating the physical stability of the colloidal system. Zeta potential was measured using the Laser-Doppler method.
(3) Measurement of drug content: The content of progerinin for the total weight of the nanosuspension was measured.
(4) Measurement of dispersibility: Dispersibility was evaluated by visual observation depending on the precipitation of the suspension.
(5) Viscosity measurement: Measurement was performed at a torsional moment of 32.9%, temperature of 25°C, probe rotation rate of 200 rpm, shear force of 13.03 dyne/cm², and shear rate of 264.0 S⁻¹ for 1 minute using a BROOKFIELD viscometer (TC-550MX-230).

**TABLE 3**

| Category | Prototype 1 | Prototype 2 | Prototype 3 | Prototype 4 | Prototype 5 |
|---|---|---|---|---|---|
| Composition (wt%) | HPMC E3 3%/ TPGS 1%/ Potassium sorbate 0.2% | HPMC E3 1%/TPGS 0.25%/ Potassium Sorbate 0.2% | HPC (SL) 1%/ DOSS 0.25%/ Potassium Sorbate 0.2% | HPC (SL) 1%/ Polysorbate 80 0.25%/ Potassium Sorbate 0.2% | PVP K30 1%/ Poloxamer 188 0.25%/ Benzoic acid 0.2% |
| D50 (Unit: nm) | 165.8 | 235.2 | 146.3 | 191.4 | N/A |
| D90 (Unit: nm) | 281.8 | 414.4 | 260.8 | 342.5 | N/A |
| PDI | 0.171 | 0.195 | 0.203 | 0.206 | N/A |
| Zete potential (Unit: mV) | -2.07 | N/A | -17.76 | -5.84 | N/A |
| API concentration of suspension (Unit: mg/mL) | 100.9 | N/A | 107.6 | N/A | N/A |
| Purity of suspension (Unit: %) | 99.9 | N/A | 99.9 | N/A | N/A |
| Impurity of suspension | - | N/A | - | N/A | N/A |
| (Unit: %) | | | | | |
| API concentration of supernatant (Unit: mg/mL) | 1.5 | N/A | 2.4 | N/A | N/A |
| Purity of supernatant (Unit: %) | 95.4 | N/A | 99.9 | N/A | N/A |
| Impurity of supernatant, (Unit: %) | 4.538 (RRT 0.963) | N/A | - | N/A | N/A |
| Flowability | good | good | good | good | Very poor |
| Viscosity (Unit: mPa·s) | 7.72 | N/A | 2.25 | 4.28 | N/A |

From Table 3, it was found that the average particle diameters (D50) of the drug particles of prototype 1 and prototype 3 were 165.8 nm and 146.3 nm, respectively with the average particle diameter of 200 nm or less satisfied, and the drug content had a value higher than 95% without the detection of impurities.

Therefore, it was possible to determine that prototypes 1 and 3 are the most suitable vehicle compositions in terms of dispersibility, stability, and effectiveness of the drug. On the other hand, due to limitations in terms of effectiveness of the drug, the prototype 4 was not selected as a suitable vehicle composition. In the case of prototype 2, the tendency that particles increase over time, made it an unsuitable candidate. In the case of Prototype 5, the formation of nanosuspensions was not possible at all.

### <Example 3> Selection of optimized excipients

To assess compatibility with excipients, progerinin was mixed with HPMC E3, TPGS, and potassium sorbate in a ratio of 1: 10, and, as a result of measuring whether the drug (API) components are effective under 40°C/75% RH condition over 2 weeks, it was found that the addition of HPMC E3, TPGS, and potassium sorbate to the progerinin drug had no effect on the drug. In particular, in the case of preservatives, the content decreases when added before milling, such that it is preferable to complete it by post-addition after milling, and the preliminary excipient compatibility studies suggested that the drugs had compatibility with excipients.

### <Example 4> Identification of stability

Stabilization studies of progerinin nanosuspensions were further conducted on prototypes 1 and 3, which showed excellent particle stability in the course of the vehicle solution selection. Specifically, evaluation was performed at 2 to 8°C, 40°C/RH 75% conditions, or 1.2 M lux exposure over a period of 3 weeks. The study on the stabilization of the progerinin nanosuspension was conducted at room temperature (25 ± 5°C, 60% RH) for 3 weeks. Characterization was conducted in relation to particle size and drug content of the progerinin nanosuspension. The results are shown in Tables 4 and 5.

**TABLE 4**

| Category | Prototype 1 HPMC E3 3%/ TPGS 1%/ Potassium sorbate 0.2% | | | | | | | |
|---|---|---|---|---|---|---|---|---|
| Condition | Initial | 2-8°C (1-week) | 2-8°C (2-week) | 2-8°C (3-week) | 40°C /RH75% (1-week) | 40°C /RH75% (2-week) | 40°C /RH75% (3-week) | 25°C.2MLux |
| D50(Unit: nm) | 165.8 | 208.3 | 208.0 | 200.0 | 194.0 | 194.2 | 195.9 | 186.3 |
| D90(Unit: nm) | 281.8 | 346.4 | 347.7 | 336.2 | 339.6 | 333.6 | 329.1 | 352.2 |
| PDI | 0.171 | 0.158 | 0.161 | 0.164 | 0.191 | 0.178 | 0.164 | 0.247 |
| Zete potential (Unit: mV) | -2.07 | N/A | N/A | N/A | N/A | N/A | N/A | N/A |
| API concentration of nanosuspension (Unit: mg/mL) | 100.9 | 108.3 | 103.5 | 99.8 | 103.1 | 99.7 | 97.6 | 103.9 |
| Purity of suspension (Unit: %) | 99.9 | 99.9 | 99.9 | 99.5 | 99.9 | 99.9 | 99.4 | 99.5 |
| Impurity of suspension (Unit: %) | - | - | - | - | - | - | - | 0.468 (RRT 1.015) |
| API concentration of supernatant (Unit: mg/mL) | 1.5 | 1.4 | 1.4 | 2.0 | 0.834 | 0.62 | 1.4 | 0.821 |
| Purity of supernatant (Unit: %**) | 99.9 | 99.9 | 99.9 | 99.5 | 99.9 | 99.9 | 95.9 | 99.9 |
| Impurity of supernatant (Unit: %) | - | - | - | RRT0.66 0.45% | - | - | RRT0.40 0.765; RRT0.43 1.6%; RRT0.52 1.7% | - |
| Flowability | good | good | good | good | good | good | good | good |
| Viscosity (Unit: mPa·s) | 7.72 | N/A | N/A | N/A | N/A | N/A | N/A | N/A |
| - N/A : No data because data cannot be measured | | | | | | | | |

**TABLE 5**

| Category | Prototype 3 HPMC (SL) 1%/ DOSS 0.25%/ Potassium Sorbate 0.2% | | | | | | | |
|---|---|---|---|---|---|---|---|---|
| Condition | Initial | 2-8 °C 1-week | 2-8 °C 2-week | 2-8 °C 3-week | 40 °C/ RH75% 1-week | 40 °C/ RH75% 2-week | 40 °C/ RH75% 3-week | 25 °C 1.2MLux |
| D50(Unit: nm) | 146.3 | 174.8 | 177.8 | 189.5 | 162.4 | 162.2 | 169.2 | 153.7 |
| D90(Unit: nm) | 260.8 | 321.3 | 330.6 | 345.3 | 287.5 | 288.7 | 302.0 | 270.4 |
| PDI | 0.203 | 0.226 | 0.234 | 0.219 | 0.199 | 0.202 | 0.204 | 0.194 |
| Zete potential(Unit: mV) | -17.76 | N/A | N/A | N/A | N/A | N/A | N/A | N/A |
| API concentration of nanosuspension (Unit: mg/mL) | 107.6 | 107.5 | 108.8 | 101.0 | 103.5 | 101.2 | 98.1 | 102.4 |
| Purity of suspension (Unit: %) | 99.9 | 99.9 | 99.9 | 99.9 | 99.9 | 99.9 | 99.9 | 99.4 |
| Impurity of suspension (Unit: %) | - | - | - | - | - | - | - | 0.597 (RRT 1.015) |
| API concentration of supernatant (Unit: mg/mL) | 2.4 | 1.3 | 1.4 | 2.0 | 0.31 | 1.3 | N/A | 0.29 |
| Purity of supernatant (Unit: %) | 99.9 | 99.9 | 99.9 | 99.9 | 99.9 | 99.9 | 99.9 | 99.9 |
| Impurity of supernatant, (Unit: %) | - | - | - | - | - | - | - | - |
| Flowability | good | good | good | good | good | good | good | good |
| Viscosity, (Unit: mPa·s) | 2.25 | N/A | N/A | N/A | N/A | N/A | N/A | N/A |
| - N/A : No data because data cannot be measured | | | | | | | | |

As shown in Table 4 and Table 5, it was found that, even under low temperatures or harsh conditions, the average particle size was 200 nm or less, ensuring stability of the nanodrug particles for more than 3 weeks.

### <Example 5> Identification of stability of optimized progerinin nanosuspension in simulated intestinal fluids (SIF) and simulated gastric fluids (SGF)

The nanosuspension of prototype 1 was prepared in the same method as in Example <2-1>, and was prepared with the components and content in Table 6 below. The dispersion stability in the simulated intestinal fluid (SIF) and simulated gastric fluid (SGF) was checked for the nanosuspension prepared as described above. The specific experimental method is as follows.

**TABLE 6**

| **Component** | **Function** | **Unit Quantity (per bottle) (Unit: g)** | **Batch Quantity (per batch) (Unit: g)** | Wt% |
|---|---|---|---|---|
| SLC-D011 (Progerinin) | Active | 1.5 | 2,000.0 | 10 |
| HPMC E3 | Suspending agent | 0.45 | 600.0 | 3 |
| TPGS | Surfactant | 0.15 | 200.0 | 1 |
| Potassium Sorbate | Preservative | 0.03 | 40.0 | 0.2 |
| Purified water | Solvent | 12.87 | 17,160.0 | 85.8 |
| Total | | 15.0 | 20,000.0 | 100 |

### a) Simulated intestinal fluid (SIF)

Monobasic potassium phosphate (6.8 gm) and sodium hydroxide (0.616 gm) were added to a 1000 ml volume flask with 250 ml distilled water, then rotated until the above components are dissolved, and then another 700 ml of distilled water was added, followed by pH measurement. The pH was adjusted to pH 6.8 +/- 0.1 by adding 0.2N sodium hydroxide or 0.2N hydrochloric acid.

### b) Simulated gastric fluids (SGF)

Sodium chloride (2 gm), 750 ml of distilled water, and 7.0 ml of concentrated hydrochloric acid were added to a 1000 ml volume flask, followed by addition of 1000 ml distilled water and mixing of ingredients by rotation. The pH of this solution was set to 1.2.

### c) Preparation of the dispersion

A translucent HDPE vial (25 ml) with an HDPE lid was added with a nanosuspension of prototype 1 in an appropriate amount. At this time, it was diluted in 15 ml of simulated gastric fluids or simulated intestinal fluids to reach a final concentration of progenin to 0.5 mg/ml. Then, after adding a dispersant, the formulation prepared above was shaken until it was completely dispersed. The vial was placed in an oil bath at 37°C until flocculation takes place. The size of the precipitated particles was measured using the Horiba-LA-910 particle analyzer. In general, the above substances were cultured in human gastric fluid on an empty stomach for approximately 3 hours.

### d) Measurement of particle diameters

In the case of measuring beads coated with bead cores including insoluble particles, the weight of the bead cores was calculated in the SIF or SGF experiment, and the bead cores were dispersed in volumes such as SIF or SGF. 120 gm of distilled water was poured into the Horiba LA-910 chamber and drained until the device was empty. Afterwards, 120 gm of distilled water was poured, and the entire volume of the cultured formulation (at 15 ml of SGF or SIF) was poured into the Horiba LA-910 chamber and the average particle diameter was measured.

**TABLE 7**

| Test | Before dilution | Diluted in SGF (1V:9V) | Diluted in SIF (1V:9V) |
|---|---|---|---|
| Appearance | White homogeneous suspension | | |
| PDI. | 0.251 | 0.186 | 0.224 |
| PSD(D50, nm) | 195.0 | 218.5 | 212.5 |
| PSD(D90, nm) | 370.5 | 379.6 | 389.6 |

As shown in Table 7, the progerinin drug (prototype 1) with the average particle diameter (D50) of 200 nm or less was maintained at 190 to 220 nm even in simulated intestinal fluids or simulated gastric fluids, and it was found that the dispersion stability and bioavailability of the drug were maintained.

### <Example 6> Preparation of capsules or sachets

### <6-1> Preparation of nanosuspension (10%, w/w)

Progerinin nanosuspension (10%, w/w) was prepared in a composition of the following Table.

**TABLE 8**

| Substance name | Unit composition (w/w, %) |
|---|---|
| C19010954-C [API, Progerinin] | 10.0 |
| HPMC E3 | 3.0 |
| TPGS | 1.0 |
| Purified water | 86.0 |
| Total | 100.0 |

First, purified water was prepared at 55°C~60°C, TPGS was added to a solution preparation vessel while stirring, and the constant temperature water bath heated to 55°C~60°C was maintained until TPGS was completely dissolved. HPMC E3 was added to the solution preparation vessel while stirring and then cooled to ambient temperature (15°C~25°C) while stirring until the solid is completely dissolved and the HPMC E3 solution becomes transparent. Progerinin (Batch Formula C19010954-C) was slowly filled into the solution preparation vessel and stirred continuously.

Parameters of the Agitator Bead Mill, a mixing and wet grinding equipment, were set as in Table 9 below. The suspension from the Agitator Bead Mill was recycled back into the solution preparation vessel, the solution preparation vessel was placed in a cooler, and milling continued until the PSD result becomes D50≤200 nm.

**TABLE 9**

| Item | MULTI LAB | MULTI LAB | ECM-AP 05 |
|---|---|---|---|
| | Suggest Parameter | | |
| Nano-suspension Batch No. | FP274601-H21001 | FP274601-H21002 | D-FP274601-H21001 |
| Theoretical Batch Size | 10.0 kg | 10.0 kg | 4.540 kg |
| API Batch No. | 20112506(CR-C19010954-CF20001) | 211021001(CR-C19010954-CF21001) | 20112506(CR-C19010954-CF20001) |
| Purpose | Used for R&D development work | Human PK production | Used for R&D demo batch sachet production |
| Milling chamber | 1.4 L | 1.4 L | 0.5 L |
| Agitator paddles | 80 mm | 80 mm | 64 mm |
| Gap size | 0.1 mm | 0.1 mm | 0.13 mm |
| Milling Speed | 0 ~ 4180 rpm | 0 ∼ 4180 rpm | 5-15 m/s |
| Total milling time | 40 h | 41.5 h | 24 h |
| Grinding media type | VHD ZrO Grinding Media, 0.3 mm | VHD ZrO Grinding Media, 0.3 mm | VHD ZrO Grinding Media, 0.3 mm |
| Grinding media weight | 4300.0 g | 4300.0 g | 1200.0 g |

### <6-2> Preparation of 250 mg and 350 mg granules

Progerinin sachet formulations were prepared in compositions of the following Table 10. To the container with the nanosuspension previously prepared, mannitol 100SD, mannitol 200SD, and sodium croscarmellose SD-711 were added. The mixture was mixed for at least 2 hours, followed by checking that the redispersant was completely dissolved. After preheating the machine and materials, the granulation solution (nozzle size: 1.0mm) was sprayed. Inlet airflow, inlet air temperature, and spray rate were adjusted to maintain proper fluidization throughout the entire spray process. When the granulation process was over, the granules were dried. LOD (measured at 105°C) was monitored at appropriate time points, and drying was stopped until LOD was 2.00% or less. After drying, the inlet air temperature was set to 25.0°C (20.0~30.0°C) and cooling was started until the product temperature becomes below 35.0°C.

### <6-3> Packaging of progerinin sachet

By adding a sweetener (sucralose), a lubricant (magnesium stearate), and a flavor (grape flavor) to the prepared granules, the sachet for R&D was filled manually, and the Sachet Filling Machine was used in the production of Human PK batches.

**TABLE 10**

| Material Description | 250 mgUnit Formula (mg/bag) | | | 350 mgUnit Formula (mg/bag) | | |
|---|---|---|---|---|---|---|
| C19010954-C | 250.0 | 250.0 | 250.0 | 350.0 | 350.0 | 350.0 |
| Hydroxypropyl Methylcellulose Methocel E3 Premium LV | 75.0 | 75.0 | 75.0 | 105.0 | 105.0 | 105.0 |
| Vitamin E Polyethylene Glycol Succinate VE TPGS | 25.0 | 25.0 | 25.0 | 35.0 | 35.0 | 35.0 |
| Croscarmellose Sodium SD-711 | 7.5 | 7.5 | 7.5 | 10.5 | 10.5 | 10.5 |
| Mannitol 100SD (filler) | | 262.6 | 262.6 | | 367.7 | 367.7 |
| Mannitol 100SD (redispersant) | | 125.0 | 125.0 | | 175.0 | 175.0 |
| Mannitol 200SD (filler) | 262.6 | | | 367.7 | | |
| Mannitol 200SD (redispersant) | 125.0 | | | 175.0 | | |
| Magnesium Stearate LIGAMED MF-2-V | 3.8 | 3.8 | 3.8 | 5.3 | 5.3 | 5.3 |
| Sucralose | 0.8 | 0.8 | 0.8 | 1.1 | 1.1 | 1.1 |
| Grape Flavor | 0.38 | 0.38 | | 0.5 | 0.5 | |
| Concord Grape Flavor 20.4564.2P PHA | | | 0.38 | | | 0.5 |
| Total | 750.0 | 750.0 | 750.0 | 1050.0 | 1050.0 | 1050.0 |
| Theoretical fill weight (mg) | 750.0 | | | 1050.0 | | |

While specific parts of the present invention have been described in detail above, it is clear for those skilled in the art that these specific descriptions are merely preferred example embodiments, and the scope of the present disclosure is not limited thereby. In other words, the substantial scope of the present disclosure is defined by the appended claims and their equivalents.

## Claims

1. A granule comprising progerinin, the granule comprising:
(a) 20 to 40 wt% of progerinin represented by the following Chemical Formula 1; (b) 5 to 15 wt% of hydroxypropyl methylcellulose (HPMC); (c) 1 to 10 wt% of D-α-tocopherol polyethylene glycol succinate (TPGS); and (d) a residual amount of additives,
wherein the progerinin is drug particles formed via wet type ball milling with an average particle diameter (D50) of 100 nm to 300 nm:

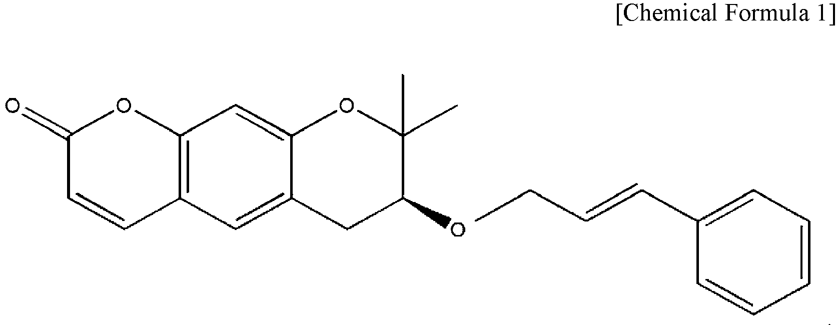

2. The granule of claim 1, wherein the average particle diameter (D50) of the progerinin is 100 nm to 200 nm.

3. The granule of claim 1, wherein the additives are one or more selected from the group consisting of excipients, binders, disintegrants, and lubricants.

4. The granule of claim 3, wherein the excipient is one or more selected from the group consisting of mannitol, starch, microcrystalline cellulose, dextrin, sodium alginate, methylcellulose, sodium carboxymethylcellulose, lactose, glucose, fructose, sodium alginate, and hydroxypropyl starch.

5. The granule of claim 3, wherein the binder is one or more selected from the group consisting of gelatin, gum arabic, glucose, ethanol, purified water, dextrin, glycerin, microcrystalline cellulose, methylcellulose, hydroxypropyl cellulose, hydroxypropyl methylcellulose, carboxymethylcellulose, sodium carboxymethylcellulose, and calcium carboxymethylcellulose.

6. The granule of claim 3, wherein the disintegrant is one or more selected from the group consisting of sodium croscarmellose, starch, hydroxypropyl starch, methylcellulose, sodium alginate, calcium citrate, sodium carboxymethylcellulose, calcium carboxymethylcellulose, silicic acid anhydride, sodium lauryl sulfate, carbonate, and dextran.

7. The granule of claim 3, wherein the lubricant is one or more selected from the group consisting of calcium stearate, magnesium stearate, sodium stearate, stearic acid, hydrogenated vegetable oil, talc, kaolin, liquid paraffin, and magnesium oxide.

8. A capsule comprising the granule according to any one of claims 1 to 7.

9. A sachet comprising the granule according to any one of claims 1 to 7, a sweetener, and a flavor.

10. A method of preparing a granule comprising progerinin, the method comprising:
(1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension;
(2) subjecting the suspension to wet type ball milling to prepare a nanosuspension; and
(3) mixing the nanosuspension with one or more additives selected from the group consisting of excipients, binders, disintegrants, and lubricants, followed by granulating in a fluid bed granulator,
wherein the progerinin in the nanosuspension of the step (2) is in the form of particles with an average particle diameter (D50) of 100 nm to 300 nm:

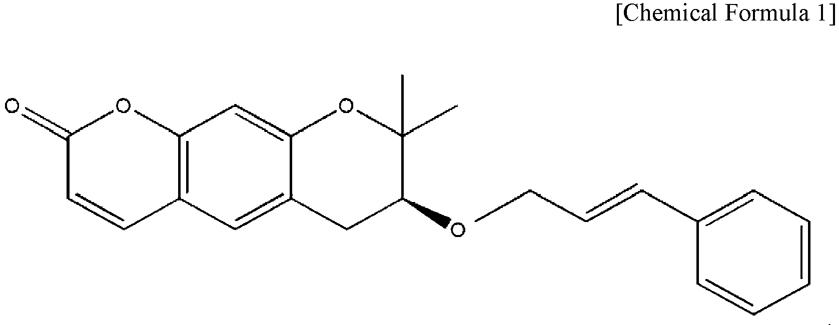

11. The method of claim 10, wherein the purified water of the step (1) has a temperature of 50 to 70°C.

12. The method of claim 10, wherein, during the wet type ball milling in the step (2), the nanosuspension is prepared by mixing and milling the suspension of the step (1) and zirconia beads in a volume ratio of 1:1 to 1:5.

13. A method of preparing a capsule comprising progerinin, the method comprising:
(1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension;
(2) subjecting the suspension to wet type ball milling to prepare a nanosuspension;
(3) mixing the nanosuspension with one or more additives selected from the group consisting of excipients, binders, disintegrants, and lubricants, followed by granulating in a fluid bed granulator; and
(4) filling the granulated granule in a capsule,
wherein the progerinin in the nanosuspension of the step (2) is in the form of particles with an average particle diameter (D50) of 100 nm to 300 nm:

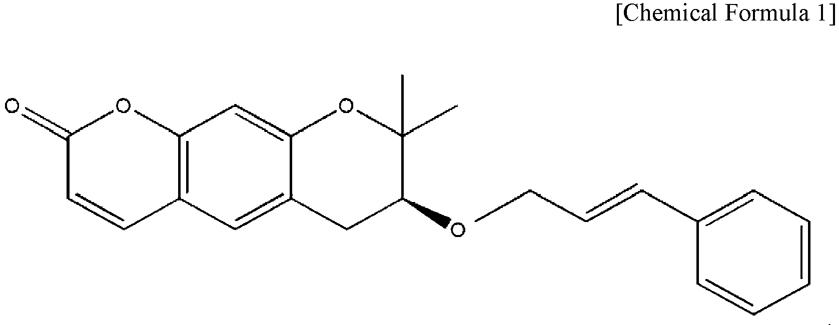

14. A method of preparing a sachet comprising progerinin, the method comprising:
(1) mixing purified water with hydroxypropyl methylcellulose (HPMC) and D-α-tocopherol polyethylene glycol succinate (TPGS) to prepare a vehicle solution and then adding and mixing progerinin represented by the following Chemical Formula 1 so as to prepare a suspension;
(2) subjecting the suspension to wet type ball milling to prepare a nanosuspension;
(3) mixing the nanosuspension with one or more additives selected from the group consisting of excipients, binders, disintegrants, and lubricants, followed by granulating in a fluid bed granulator; and
(4) adding a sweetener and a flavor to the granulated granule and then filling the granule into a sachet,
wherein the progerinin in the nanosuspension of the step (2) is in the form of particles with an average particle diameter (D50) of 100 nm to 300 nm:
